# EUROPEAN PATENT SPECIFICATION

(11) **EP 1 973 577 B1**
(45) Date of publication and mention of the grant of the patent: **01.08.2012**
(21) Application number: 06835758.1
(22) Date of filing: 15.12.2006
(51) Int. Cl.: A61L 2/06, A61M 16/00

(54) **BREATHING ASSISTANCE APPARATUS WITH THERMAL DISINFECTION CONDUIT**
GERÄT ZUR UNTERSTÜTZUNG DER ATMUNG MIT LEITUNG ZUR THERMISCHEN DESINFEKTION
APPAREIL D'ASSISTANCE RESPIRATOIRE AVEC LIGNE POUR LA DESINFECTION THERMIQUE

(30) Priority: 15.12.2005 NZ 54416905
(43) Date of publication of application: 01.10.2008
(73) Proprietor: Fisher & Paykel Healthcare Limited, East Tamaki Auckland 2013 (NZ)
(72) Inventor: O'DONNELL, Kevin, Peter C/-Fisher & Paykel Healthcare Limited, Auckland, 0630 (NZ); PAYTON, Matthew, Jon, Auckland, 1072 (NZ); QUILL, Christopher, Simon, James, Auckland, 2010 (NZ); KRAMER, Martin, Paul, Friederich, Auckland, 1051 (NZ); HAWKINS, Peter, Geoffrey, Auckland, 1051 (NZ); DAKEN, Reena, Auckland, 2102 (NZ)
(74) Representative: Hoarton, Lloyd Douglas Charles
(86) International application number: PCT/NZ2006/000330
(87) International publication number: WO 2007/069922

(56) References cited:
- WO-A-2004/026382
- WO-A-2005/011785
- WO-A1-2006/126900
- WO-A2-2005/021076
- JP-A- 04 352 965
- US-A- 5 537 996
- US-A1- 2002 124 847
- US-B1- 6 283 132
- US-B1- 6 523 538
- DATABASE WPI Week 199746 Thomson Scientific, London, GB; AN 1997-497460 XP002534023 & JP 09 234247 A (NICHI KISO YSI KK) 9 September 1997 (1997-09-09)

## Description

### BACKGROUND OF THE INVENTION

### Field of the Invention

This invention relates to a gases supply and gases humidification apparatus that can be disinfected and reused for different patients. The invention also relates to a method for disinfecting apparatus parts that extend the life of these parts for use by a single patient.

### Summary of the Prior Art

A number of methods are known in the art for assisting a patient's breathing. Continuous Positive Airway Pressure (CPAP) involves the administration of air under pressure to a patient, usually by a nasal mask. It is used in the treatment of snoring and Obstructive Sleep Apnoea (OSA), a condition characterised by repetitive collapse of the upper airway during inspiration. Positive pressure splints the upper airway open, preventing its collapse. Treatment of OSA with nasal CPAP has proven to be both effective and safe, but CPAP is difficult to use and the majority of patients experience significant side effects, particularly in the early stages of treatment.

CPAP is also commonly used for patients with a variety of respiratory illnesses, including Chronic Obstructive Pulmonary Disease (COPD).

Upper airway symptoms adversely affect treatment with CPAP. Mucosal drying is uncomfortable and may awaken patients during the night. Rebound nasal congestion commonly occurs during the following day, simulating a viral infection. If untreated, upper airway symptoms adversely affect rates of CPAP use.

Increases in nasal resistance may affect the level of CPAP treatment delivered to the pharynx, and reduce the effectiveness of treatment. An individual pressure is determined for each patient using CPAP and this pressure is set at the patient interface. Changes in nasal resistance affect pressure delivered to the pharynx and if the changes are of sufficient magnitude there may be recurrence of snoring or airway collapse or reduce the level of pressure applied to the lungs. Such symptoms can also occur in a hospital environment where a patient is on a respirator. Typically in such situations the patient is intubated. Therefore the throat tissue may become irritated and inflamed causing both distress to the patient and possible further respiratory problems.

A number of methods may be employed to treat such upper airway symptoms, including pharmacological agents to reduce nasal disease, or heating the bedroom. One most commonly employed method is humidification of the inspired air using an in line humidifier. Two types of humidifier are currently used. Cold pass-over humidifiers rely on humidifying the air through exposure to a large surface area of water. While they are cheap, the humidity output is low at high flows, typically 2 to 4 mg\L absolute humidity at flows above 25L/min. The output is insufficient to prevent mucosal drying. Heated water bath humidifiers are more efficient, and produce high levels of humidity even at high flow rates. They are effective at preventing upper airway mucosal drying, prevent increases in nasal resistance, and are the most reliable means of treating upper airway symptoms.

Oxygen is the most common drug prescribed to hospitalized patients. The delivery of oxygen via nasal cannula or facemask is of benefit to a patient complaining of breathlessness. By increasing the fraction of inspired oxygen, oxygen therapy reduces the effort to breathe and can correct resulting hypoxia (a low level of oxygen in the tissues).

The duration of the therapy depends on the underlying illness. For example, postoperative patients may only receive oxygen while recovering from surgery while patients with COPD require oxygen 16 to 18 hours per day.

Currently greater than 16 million adults are afflicted with COPD, an umbrella term that describes a group of lung diseases characterized by irreversible airflow limitation that is associated mainly with emphysema and chronic bronchitis, most commonly caused by smoking over several decades. When airway limitation is moderately advanced, it manifests as perpetual breathlessness, without physical exertion. Situations such as a tracheobronchial infection, heart failure and also environmental exposure can incite an exacerbation of COPD that requires hospitalization until the acute breathlessness is under control. During an acute exacerbation of COPD, the patient experiences an increase in difficulty of breathing (dyspnea), hypoxia, and increase in sputum volume and purulence and increased coughing.

Oxygen therapy provides enormous benefit to patients with an acute exacerbation of COPD who are hypoxic, by decreasing the risk of vital organ failure and reducing dyspnea. The major complication associated with oxygen therapy is hypercapnia (an elevation in blood carbon dioxide levels) and subsequent respiratory failure. Therefore, the dose of oxygen administered can be critical and must be precisely known.

To accurately control the oxygen dose given to a patient, the oxygen-enriched gas must exceed the patient's peak inspiratory flow to prevent the entrainment of room air and dilution of the oxygen. To achieve this, flows of greater than 20 L/min are common. Such flows of dry gases cause dehydration and inflammation of the nasal passages and airways if delivered by nasal cannula. To avoid this occurrence, a heated humidifier is used.

The majority of systems that are used for oxygen therapy or merely delivery of gases to a patient consists of a gases supply, a humidifier and conduit. Interfaces include facemasks, oral mouthpieces, tracheostomy inlets and nasal cannula, the latter having the advantage of being more comfortable and acceptable than a facemask.

A group of patients who would benefit from humidification therapy are patients who have mucociliary clearance deficiencies. These patients often have purulent mucus and are susceptible to infections from pathogens.

Heated humidified air with an abundance of water particles is an ideal medium to harbour disease carrying pathogens. Consequently, considerable design expertise has been required to provide the market with active pass-over humidifiers that deliver water molecules, in gas phase only, so that it is not possible for disease pathogens to be carried in air to the patient. Water that condenses on the inner surfaces of the breathing circuit or conduit at the end of a treatment session may harbour pathogens that would be delivered to the patient next time they use the device. This is particularly the case with humidification therapies where patients are receiving body temperature fully saturated air.

In hospital environments or similar it is often not possible for gases supply devices, such as devices that deliver CPAP and include a humidifier, to be used by multiple patients. If devices were to be used in this manner all parts, from the humidification chamber to and including the patient interface, must be disposed of or cleaned to a high standard of disinfection in between use by different patients. Often CPAP devices and humidifiers are provided in an integrated unit, such as the Sleepstyle™ 600 series CPAP device of Fisher & Paykel Healthcare Limited. This CPAP device is predominantly used for home use by an individual. This device has internal tubing from the outlet of the humidification chamber that is difficult to disinfect. As these devices are difficult to disinfect they are often not used in settings such as hospitals or clinics where multiple patients will use the device.

In the home use situation when oxygen therapy and CPAP devices are used by a single patient the lifespan of the breathing tube and patient interface is determined by the mechanical lifespan of the parts and the build up of microbial pathogens on the breathing gases path of these parts. Often it is hard to lower microbial contamination on the breathing gas surfaces of these parts.

US2002/012847 discloses a humidified gases delivery apparatus comprising a humidifier connectable to a heated gases conduit.

JP9234247 discloses a heatable gases conduit attachable to a humidifier provided with temperature and moisture sensors.

US5537996 discloses a heated respiratory humidifier conduit.

WO2004/026382 and W02005/011785 disclose a breathing assistance apparatus having the pre-characterising features of the claims.

### DISCLOSURE OF THE INVENTION

It is an object of the present invention to provide a breathing assistance apparatus which goes some way to overcoming the abovementioned disadvantages or which at least provides the public or industry with a useful choice.

Accordingly in a first aspect the present invention consists in a breathing assistance apparatus according to claim 1.

The present invention further provides a method according to claim 4; and a breathing assistance apparatus and method according to the dependent claims.

### BRIEF DESCRIPTION OF THE DRAWINGS

A preferred form of the present invention will now be described with reference to the accompanying drawings.
**Figure 1** is an illustration of the breathing assistance apparatus that may utilise the method of disinfecting of the present invention.
**Figure 2** is an exploded view of an elbow connection conduit of the breathing assistance apparatus of the present invention.
**Figure 3** is a front view of a gases supply and humidifier apparatus with a hot gases tube (disinfection conduit) and a filter connection of the present invention.
**Figure 4** is a front view of the gases supply and humidifier apparatus of Figure 3 without the disinfection conduit and ready for use by a patient.
**Figure 5** is a perspective view of a breathing assistance apparatus with a filter over a gases return of the breathing assistance apparatus.
**Figure 6** is a close up view of the gases return with the filter, in particular, the filter cover and a projection that extends within the gases return.
**Figure 7** is a front view of the gases supply and humidifier apparatus showing the gases return and the temperature sensor residing within it.

### DETAILED DESCRIPTION OF THE PREFERRED EMBODIMENTS

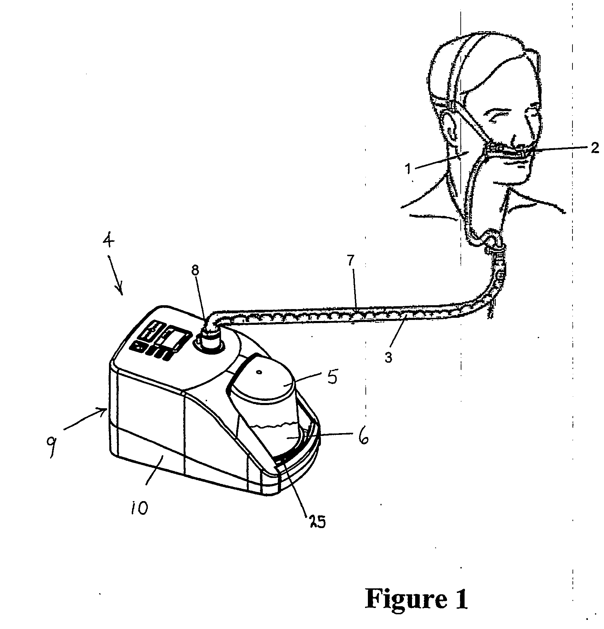

The present invention provides a breathing assistance apparatus that has a convenient and effective method of cleaning internal conduits inside the apparatus. As shown in Figure 1, the flow of gases passes in sequence through a gases supply means or flow driver (such as, a blower, fan or compressor), humidification chamber, heated delivery circuit and patient interface.

Gases are passed to the patient by way of a patient interface 2. The patient interface used with the apparatus of the present invention may be a full-face mask, nasal mask, nasal cannula, oral mouthpiece or tracheostomy connection, but the description below and figures disclose the use of a nasal cannula.

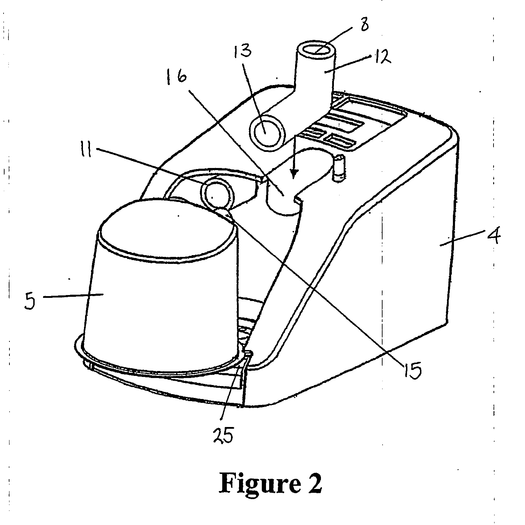

With reference to Figure 1 the breathing assistance apparatus of the present invention is shown in which a patient 1 is receiving humidified and pressurised gases through a nasal cannula 2. The cannula 2 is connected to a gases transportation pathway or inspiratory conduit 3 that in turn is connected to an integrated gases supply (blower) and humidifying device 4 (including a humidification chamber 5). In the preferred embodiment of the blower-humidifying device 4, the gases supply or blower is combined in one housing with the humidifier and humidification chamber. The humidification chamber 5 extends out from the housing 10 and is capable in use of being removed and replaced (by a slide on movement, such as that described in WO04024429 of Fisher & Paykel Healthcare Limited, the contents of which are incorporated by reference) by the patient or other user. Also, the gases supply outlet port 11 (see Figure 2) that feeds the inlet to the humidification chamber 5 is internal within the housing 10. It must be appreciated that the embodiment described above in relation to the housing and the figures merely illustrates one form of the housing of the integrated gases supply and humidifying device.

The inspiratory conduit 3 (see Figure 1) is connected to the patient outlet 8 of a humidification chamber 5 that contains a volume of water 6. Inspiratory conduit 3 preferably contains heating means or heater wires 7 that heat the walls of the conduit to reduce condensation of humidified gases within the conduit and the patient interface (e.g. nasal cannula 2) such the conduit as described in WO04026382 of Fisher & Paykel Healthcare Limited, the contents of which are incorporated by reference.

The humidification chamber 5 is preferably formed from a plastics material and may have a highly heat conductive base (for example an aluminium base) that is in direct contact with a heater plate 25. The device 4 is provided with control means or an electronic controller that may comprise a microprocessor based controller executing computer software commands stored in associated memory. The controller receives input from sources such as user input means or dial (not shown) through which a user of the device 4 may, for example, set a predetermined required value (preset value) of humidity or temperature of the gases supplied to patient 1.

In response to the user set humidity or temperature value input via dial (or buttons) and other possible inputs such as internal sensors that sense gases flow or temperature, or by parameters calculated in the controller, the controller determines when (or to what level) to energise heater plate to heat the water 6 within humidification chamber 5. As the volume of water 6 within humidification chamber 5 is heated, water vapour begins to fill the volume of the chamber above the water's surface and is passed out of the humidification chamber outlet 8 with the flow of gases (for example air) provided from a blower part of the device that has entered the device 4 through an inlet 9 on the back of the device 4.

The gases supply within the device 4 is preferably a variable speed pump or fan that draws air or other gases through the blower inlet 9. The speed of variable speed pump or fan is preferably controlled by the control means or electronic controller described above in response to inputs entered into the device 4 by the user.

### Disinfection of the Device

A partially exploded view of a gases supply and humidification device 4 is shown in Figure 2. The device 4 has an elbowed connection conduit 12 that has an inlet end 13, which is the gases return in the housing 4, and a patient outlet end 8, that is the patient outlet of the housing 4. The conduit 12 is provided to receive humidified gases from the humidification chamber 5 and as such the inlet end 13 is connected to the outlet 15 of the humidification chamber. The humidified gases are directed from the conduit's patient outlet end 8 into the breathing conduit 3 (see Figure 1) for delivery to a patient. It is preferred that the elbowed connection conduit 12 is permanently fixed in place in the housing 4.

There is a requirement for multiple users to use these devices as they will be used in hospitals, sleep laboratories or leased by home care companies and hospitals for short term home users.

To use such a gases supply and humidification device 4 on multiple patients the elbow conduit 12 integrated into the device must have a high level disinfection process carried out between different patients using it. For ease of use, even if the elbow conduit 12 can be removed, and to avoid dismantling and potential damage to the internal parts of the device 4 it is preferred that the elbow is disinfected in situ. This is because it is often impractical to remove the elbow, as it may have electrical connectors and the like, and clean it and reconnect it. Furthermore, the high labour content and skill level required would make removing the elbow an unreliable cleaning method and may make the device more unreliable.

Other parts of the device 4, such as the gas supply outlet 11, may also need high level disinfection between patients and could benefit from the present invention.

Currently high level disinfection is performed either by thermal or chemical process. Thermal disinfection is normally carried out by submersion in hot water or steam and chemical disinfection by submersion in instrument grade disinfectants. These processes have disadvantages for high level disinfection of devices such as the gases supply and humidification device 4 of the present invention as it cannot be submersed; steam sterilized or easily chemically disinfected.

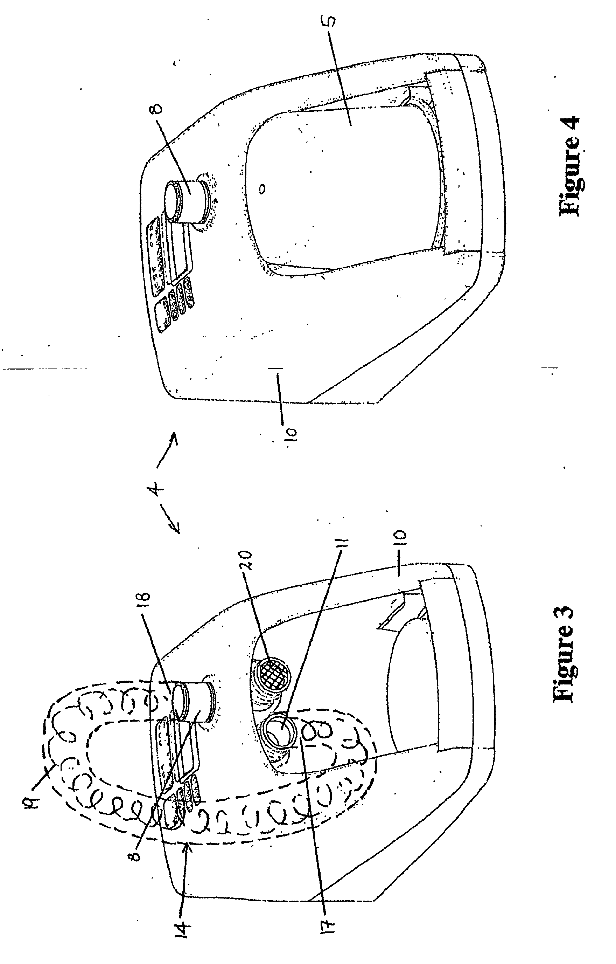

A further option for thermal disinfection is hot dry air. The present invention is the supply of a heated gases tube (disinfection conduit) for use with a device 4 to allow in particular for disinfection of the elbow conduit 12. A disinfection conduit 14 is shown in Figure 3. To disinfect the elbow conduit 12 a first end 17 of the disinfection conduit 14 is connected to the gases supply outlet 11 and a second end 18 to the patient outlet end 8 of the elbow conduit 12. The disinfection conduit 14 has a heating element 19 within, through out or about the walls of the disinfection conduit 14. For example, the disinfection conduit 14 may be a section of tubing as described in WO04026382 of Fisher & Paykel Healthcare Limited with appropriate connectors on both ends of the tube to enable connection to the elbow conduit 12 and the gases supply outlet 11.

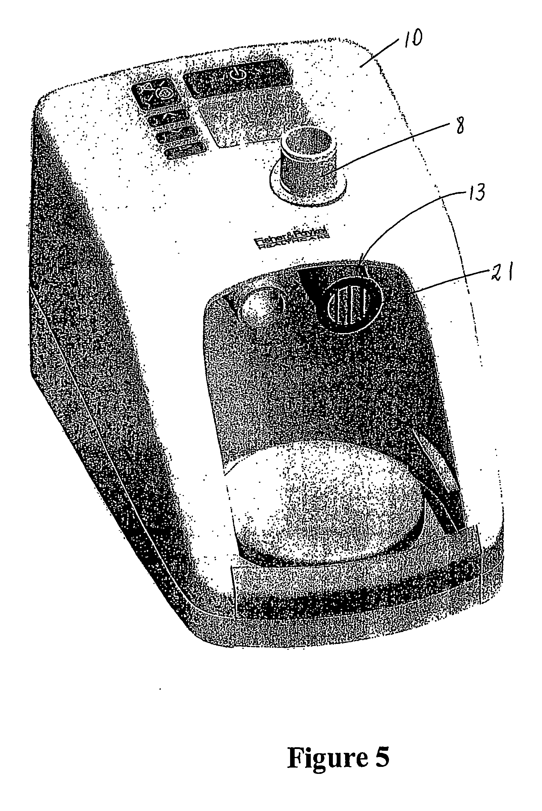

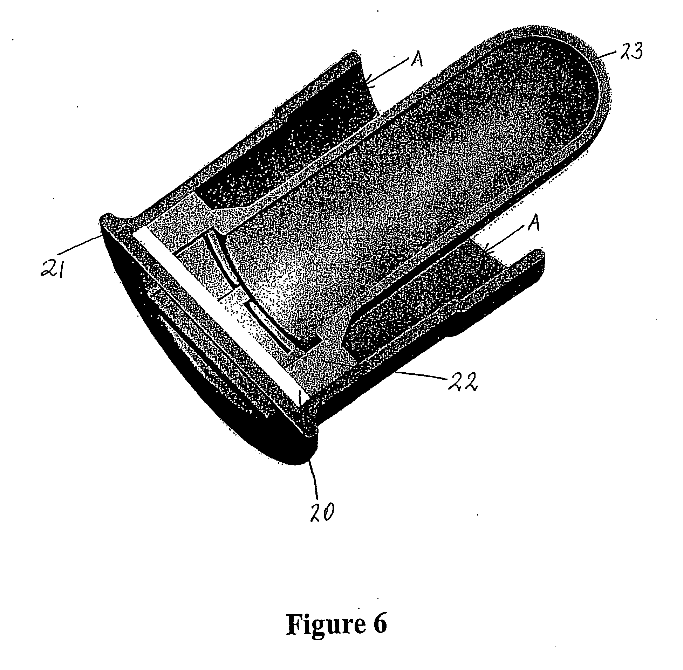

It is preferred that a removable exhaust gases filter 20 is placed on the inlet end 13 of the elbow conduit 12. This filter 20 is shown in more detail in Figures 5 and 6. In Figure 5, a filter housing 21 can be placed about the inlet end 13. The filter housing 21 is preferably made of a plastics material and can be simply removably attached to the inlet end 13, and remains on the end 13 by a friction fit. A preferably circular piece of filter material (not shown in this figure), sits inside the filter housing 21 and therefore occludes the inlet end 13, such that when in use with the disinfection conduit as described and shown with reference to Figure 3, gases exiting the inlet end 13 (patient return 13) are filtered before they exit to the ambient surroundings.

Referring to Figure 6, the filter housing 21 is shown in further detail. Preferably a projection 22 is provided with the filter 20 and housing 21. This tubular projection 22 sits within the inlet end 13 and the filter housing 21 sits about the projection 22 and over the end of the inlet 13. The projection 22 has the purpose of increasing the gases velocity at the point where heated gases are exiting the inlet end 13 (when the disinfection conduit 14 is in place as described above and in use). This has the effect of maximising the exhaust temperature of the exiting gases and minimising the temperature drop at the exit point. This ensures there is a high level of disinfection throughout the entire elbow connection conduit 12. In use, the heated gases flow around the spherical end 23 of the projection 22 down the sides of the tubular inlet end (in the direction of arrows A) through apertures (not shown) in the projection 22 through the filter material 20 and past the filter housing 21 into the ambient surroundings.

When the disinfection conduit 14 is connected between the gases supply outlet 11 and the patient outlet 8 as described above, the device 4 may be put into a cleaning mode whereby dry gases, such as air, are circulated through the disinfection conduit 14 and elbow conduit 12. It is preferable that the circulated gases are heated by the disinfection conduit to a temperature of 80°C. However, a temperature between 60°C and 90°C may be suitable.

The connectors at both ends 17, 18 of the disinfection conduit 14 preferably have pneumatic fittings. Preferably the second end 18 of the disinfection conduit 14 has an electrical connection such that the heating element 19 within the conduit 14 can be supplied from the device 4 with power. In other forms of the device both ends 17, 18 may have electrical and pneumatic connections.

The device 4, and in particular, the controllers within the device 4 that control the heating of the heater plate as described above, is capable of controlling the temperature of the heating element 19.

The device 4 preferably has a setting that causes the controllers to provide a flow of gases, for example, between 1 and 50 litres per minute, to the disinfection conduit 14 and simultaneously provide power to the heating element 19, such that the gases circulating through the conduit 14 increase in temperature to 80°C. The controller then maintains the power to the heating element 19 maintaining a predetermined temperature inside the conduit 12 for a period of time, preferably 20 minutes, in order for the heated dry gases to disinfect the elbow conduit 12.

Therefore in use, after a patient has used the device 4 of the present invention, and before the next patient uses it, the patient, hospital staff or home care supplier can connect the disinfection conduit 14 to the device 4, as described above, and put the device 4 into a cleaning mode. As an example, a person might put the device into a cleaning mode by activating a button on the device that causes the controllers to provide power to the heating element and gases to the disinfection conduit at a predetermined temperature over a set period of time. In the preferred method of disinfecting the device, it is preferred that the surface temperatures inside the elbow conduit reaches a minimum of 80°C for a time period of 20 minutes and the flow provided for circulation through the elbow conduit is approximately 10 litres per minute. However, other appropriate circulating flows between 1 and 50 litres per minute and other appropriate time periods and temperatures may be used.

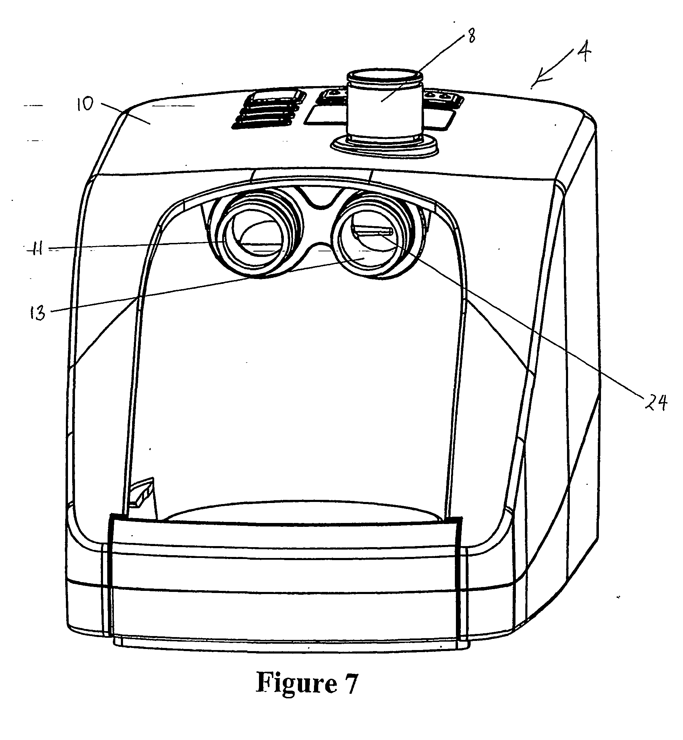

In the preferred form of the present invention the elbow conduit 12 preferably has a temperature sensor 24 within it, for example, as shown in Figure 7. However, in other forms of the invention, the elbow conduit may not have such a sensor.

In the preferred form this sensor measures the temperature of gases travelling through the conduit 12. In particular, in the preferred form of the method of disinfecting, at the start of the disinfection process, the controller or electronics within the device 4 performs some checks to ensure the disinfection conduit is correctly connected to the device 4. Firstly, a check is performed to determine that there is a heated gases flow through the elbow conduit 12. When the gas flow supplied by the device 4 and heater element 19 of the disinfection conduit 14 are both turned on, the heated gases flow is measured by the internal temperature sensor 24 inside the elbow conduit 12. After a predetermined period the heater element 19 is turned off with the gases flow of the device 4 left on. After a further predetermined period, the temperature inside the elbow conduit 12 is again measured by the temperature sensor 24. It is expected that the temperature will have dropped between measurements. This proves there is a fluid connection of gases supply connecting the flow source to the elbow conduit 12.

If a rise in temperature and a fall in temperature between measurements is not detected by the sensor 24 an error alarm will be indicated on or audibly relayed by the device, indicating the disinfection conduit 14 is not connected to the device 4 correctly.

Knowing the gases flow, ambient temperature outside the device 4 (as the device 4 has an ambient air temperature sensor incorporated within it) and the time period, in which the heating element 19 is powered, an expected temperature can be calculated for the heating and non-heating periods described above. The expected temperature can then be compared with the actual measured temperature from the temperature sensor. If these match, then the checks are complete and the disinfection conduit 14 and heating element 19 are connected and working correctly. The disinfection method, cleaning mode, as described above is then started.

In another embodiment of the present invention, the disinfection conduit may be used on a blower and humidifying device that does not have an internal temperature sensor inside the elbow conduit. In this embodiment a predetermined gases flow is caused to pass through the disinfection conduit, and a predetermined power is applied to the heating element within the disinfection conduit. The ambient conditions are known. The predetermined gases flow and predetermined power applied are determined such that the internal surface temperatures inside the elbow conduit will exceed the temperatures required for high level disinfection for a range of ambient conditions. The predetermined temperature and power are preferably determined from testing and the like.

The method of disinfection described above has been validated by an independent laboratory, Toxikon Corporation of Bedford, MA, USA.

The method of disinfection as described above is automated so minimal training is required to disinfect the elbow conduit and it is not necessary to dismantle the gases supply and humidification device, therefore, inadvertent damage to the internal parts of the device is avoided.

Also, during the disinfection period the internal surface temperature of the elbow conduit is continuously monitored so each disinfection cycle can be validated and a disinfection completed symbol shown on the display at the completion of the process. Alternatively, if the disinfection cycle was not validated a failed disinfection symbol can be displayed to alert a user or operator.

## Claims

1. A breathing assistance apparatus (4) adapted to deliver humidified gases to a patient (1) comprising:
a housing (10),
a gases supply within said housing (10),
a gases supply outlet port (11) in said housing (4), in fluid connection with said gases supply and adapted to in use make fluid connection with an inlet of a humidifier (5) in order to supply gases to said humidifier (5), an outlet (15) in said humidifier (5),
a connection conduit (12) having an inlet end (13) and a patient outlet (8) on said housing,
said inlet end (13) adapted to make fluid connection with a humidifier outlet (15) in order to receive humidfied gases from said humidifier (5),
said patient outlet (8) in fluid connection or adapted to make fluid connection with a breathing conduit (3) for delivery of humidified gases to said patient (1); and
a controller
***characterised in that** said breathing assistance apparatus further comprises*
a disinfection conduit (14) including a heating element (19) adapted for connection between said gases supply outlet port (11) and said patient outlet (8), and
said controller is capable of controlling the temperature of the heating element (19).

2. A breathing assistance apparatus according to claim 1 wherein said inlet end (13) further includes a temperature sensor (24) adapted to sense the temperature of gases flowing through said inlet end (13).

3. A breathing assistance apparatus according to claim 1 or 2 wherein said apparatus further includes a filter (20) connectable to said inlet end (13) to filter gases exiting said inlet end (13).

4. A method for disinfecting a breathing assistance apparatus (4) according to any preceding claim
said method comprising the steps of:
connecting the disinfection conduit (14), between said gases supply outlet port (11) and said patient outlet (8),
providing a flow of gases from said gases supply outlet port (11) through said gases disinfection conduit (14) for a predetermined period of time,
supplying power to said heating element (19) for said predetermined period of time to heat said disinfection conduit (14) up to a predetermined temperature so as to heat gases passing through said gases disinfection conduit (14).

5. A method of disinfecting a breathing assistance apparatus (4) according to claim 4 wherein said predetermined period of time is between 2 and 60 minutes.

6. A method of disinfecting a breathing assistance apparatus (4) according to claim 4 wherein said predetermined period of time is approximately 30 minutes.

7. A method of disinfecting a breathing assistance apparatus (4) according to any one of claims 4 to 6 wherein said predetermined temperature is between 60 and 90 degrees Celsius.

8. A method of disinfecting a breathing assistance apparatus (4) according to any one of claims 4 to 6 wherein said predetermined temperature is approximately 80 degrees Celsius.

9. A method of disinfecting a breathing assistance apparatus (4) according to any one of claims 4 to 8 wherein said flow of gases is provided at a rate of between 1 and 50 litres per minute.

10. A method of disinfecting a breathing assistance apparatus (4) according to any one of claims 4 to 8 said flow of gases is provided at a rate of approximately 10 litres per minute.

## Patentansprüche

1. Atmungsunterstützungsvorrichtung (4), die für das Zuführen eines befeuchteten Gases zu einem Patienten (1) ausgebildet ist, die Folgendes aufweist:
ein Gehäuse (10);
eine Gasversorgung innerhalb des Gehäuses (10);
eine Gasversorgungs-Austrittsöffnung (11) in dem Gehäuse (4), die in Fluidverbindung mit der Gasversorgung ist, und dafür ausgelegt ist, bei der Anwendung eine Fluidverbindung mit einem Einlass eines Befeuchters (5) herzustellen, um den Befeuchter (5) mit Gas zu versorgen,
einen Auslass (15) in dem Befeuchter (5),
eine Verbindungsleitung (12) mit einem Einlassende (13) und einem Patientenauslass (8) an dem Gehäuse, wobei das Einlassende (13) dafür ausgelegt ist, eine Fluidverbindung mit einem Befeuchteraustritt (15) herzustellen, um befeuchtete Gase von dem Befeuchter (5) zu empfangen,
wobei der Patientenauslass (8) in Fluidverbindung ist mit oder dafür ausgelegt ist, eine Fluidverbindung mit einer Atmungsleitung (3) zum Zuführen befeuchteter Gase zu dem Patienten (1) herzustellen; und
eine Steuerungseinheit,
**dadurch gekennzeichnet, dass** die Atmungsunterstützungsvorrichtung ferner Folgendes aufweist:
eine Desinfektionsleitung (14), die ein Heizelement (19) enthält, die für die Verbindung zwischen der Gasversorgungs-Austrittsöffnung (11) und dem Patientenauslass (8) ausgelegt ist, und
wobei die Steuerungseinheit zur Regelung der Temperatur von dem Heizelement (19) in der Lage ist.

2. Atmungsunterstützungsvorrichtung nach Anspruch 1, wobei das Einlassende (13) ferner einen Temperaturfühler (24) enthält, der dafür ausgelegt ist, die Temperatur der Gase zu fühlen, die durch das Einlassende (13) strömen.

3. Atmungsunterstützungsvorrichtung nach Anspruch 1 oder 2, wobei die Vorrichtung ferner einen Filter (20) enthält, der an das Einlassende (13) anschließbar ist, um die Gase, die aus dem Einlassende (13) ausströmen, zu filtern.

4. Verfahren zum Desinfizieren einer Atmungsunterstützungsvorrichtung (4) nach einem der vorangehenden Ansprüche,
wobei das Verfahren die folgenden Schritte umfasst:
Anschließen der Desinfektionsleitung (14) zwischen der Gasversorgungs-Austrittsöffnung (11) und dem Patientenauslass (8),
Bereitstellung einer Strömung von Gasen aus der Gasversorgungs-Austrittsöffnung (11) durch das die Gas-Desinfektionsleitung (14) für eine vorgegebene Zeitspanne,
Versorgung des Heizelements (19) mit Strom für die vorgegebene Zeitspanne, um die Desinfektionsleitung (14) auf eine festgelegte Temperatur derart zu erwärmen, dass die Gase, welche durch die Gas-Desinfektionsleitung (14) strömen, erwärmt werden.

5. Verfahren zum Desinfizieren einer Atmungsunterstützungsvorrichtung (4) nach Anspruch 4, wobei die vorgegebene Zeitspanne zwischen 2 und 60 Minuten liegt.

6. Verfahren zum Desinfizieren einer Atmungsunterstützungsvorrichtung (4) nach Anspruch 4, wobei die vorgegebene Zeitspanne annähernd 30 Minuten beträgt.

7. Verfahren zum Desinfizieren einer Atmungsunterstützungsvorrichtung (4) nach einem der Ansprüche 4 bis 6, wobei die festgelegte Temperatur zwischen 60 und 90 Grad Celsius liegt.

8. Verfahren zum Desinfizieren einer Atmungsunterstützungsvorrichtung (4) nach einem der Ansprüche 4 bis 6, wobei die festgelegte Temperatur annähernd 80 Grad Celsius beträgt.

9. Verfahren zum Desinfizieren einer Atmungsunterstützungsvorrichtung (4) nach einem der Ansprüche 4 bis 8, wobei die Strömung von Gasen mit einer Geschwindigkeit von zwischen 1 und 50 Liter pro Minute bereitgestellt wird.

10. Verfahren zum Desinfizieren einer Atmungsunterstützungsvorrichtung (4) nach einem der Ansprüche 4 bis 8, wobei die Strömung von Gasen mit einer Geschwindigkeit von annähernd 10 Liter pro Minute bereitgestellt wird.

## Revendications

1. Appareil (4) d'assistance respiratoire conçu pour distribuer des gaz humidifiés à un patient (1), comportant :
une enceinte (10),
une arrivée de gaz à l'intérieur de ladite enceinte (10),
un orifice (11) de sortie d'arrivée de gaz dans ladite enceinte (10), en liaison fluide avec ladite arrivée de gaz et conçu pour établir, en cours d'utilisation, une liaison fluide avec une entrée d'un humidificateur (5) afin d'amener des gaz audit humidificateur (5), une sortie (15) dans ledit humidificateur (5),
un conduit (12) de liaison doté d'une extrémité (13) d'entrée et d'une sortie (8) pour patient sur ladite enceinte,
ladite extrémité (13) d'entrée étant conçue pour établir une liaison fluide avec une sortie (15) d'humidificateur afin de recevoir des gaz humidifiés en provenance dudit humidificateur (5),
ladite sortie (8) pour patient étant en liaison fluide ou conçue pour établir une liaison fluide avec un conduit respiratoire (3) pour distribuer des gaz humidifiés audit patient (1) ; et
un régulateur
**caractérisé en ce que** ledit appareil d'assistance respiratoire comporte en outre
un conduit (14) de désinfection comprenant un élément chauffant (19) conçu pour être raccordé entre ledit orifice (11) de sortie d'arrivée de gaz et ladite sortie (8) pour patient, et
ledit régulateur étant capable de réguler la température de l'élément chauffant (19).

2. Appareil d'assistance respiratoire selon la revendication 1, ladite extrémité (13) d'entrée comprenant en outre un capteur (24) de température conçu pour détecter la température de gaz s'écoulant à travers ladite extrémité (13) d'entrée.

3. Appareil d'assistance respiratoire selon la revendication 1 ou 2, ledit appareil comprenant en outre un filtre (20) susceptible d'être relié à ladite extrémité (13) d'entrée pour filtrer des gaz quittant ladite extrémité (13) d'entrée.

4. Procédé de désinfection d'un appareil (4) d'assistance respiratoire selon l'une quelconque des revendications précédentes,
ledit procédé comportant les étapes consistant à raccorder le conduit (14) de désinfection entre ledit orifice (11) de sortie d'arrivée de gaz et ladite sortie (8) pour patient,
assurer un écoulement de gaz en provenance dudit orifice (11) de sortie d'arrivée de gaz à travers ledit conduit (14) de désinfection pendant une durée prédéterminée,
alimenter en énergie ledit élément chauffant (19) pendant ladite durée prédéterminée pour chauffer ledit conduit (14) de désinfection jusqu'à une température prédéterminée de façon à chauffer des gaz passant à travers ledit conduit (14) de désinfection de gaz.

5. Procédé de désinfection d'un appareil (4) d'assistance respiratoire selon la revendication 4, ladite durée prédéterminée étant comprise entre 2 et 60 minutes.

6. Procédé de désinfection d'un appareil (4) d'assistance respiratoire selon la revendication 4, ladite durée prédéterminée étant d'environ 30 minutes.

7. Procédé de désinfection d'un appareil (4) d'assistance respiratoire selon l'une quelconque des revendications 4 à 6, ladite température prédéterminée étant comprise entre 60 et 90 degrés Celsius.

8. Procédé de désinfection d'un appareil (4) d'assistance respiratoire selon l'une quelconque des revendications 4 à 6, ladite température prédéterminée étant d'environ 80 degrés Celsius.

9. Procédé de désinfection d'un appareil (4) d'assistance respiratoire selon l'une quelconque des revendications 4 à 8, ledit écoulement de gaz étant assuré à un débit compris entre 1 et 50 litres par minute.

10. Procédé de désinfection d'un appareil (4) d'assistance respiratoire selon l'une quelconque des revendications 4 à 8, ledit écoulement de gaz étant assuré à un débit d'environ 10 litres par minute.
